# EUROPEAN PATENT APPLICATION

(11) **EP 4 807 004 A1**
(43) Date of publication of application: **16.09.2026**
(21) Application number: 24888737.4
(22) Date of filing: 06.11.2024
(51) Int. Cl.: C12N 15/12, C07K 14/72, C12N 5/10, C12N 15/53, C12Q 1/06, C12Q 1/66, C12Q 1/6897, G01N 33/50

(54) **REPORTER CELL FOR TSH ACTIVITY MEASUREMENT**

(30) Priority: 07.11.2023 JP 2023190104
(71) Applicant: Cosmic Corporation Co., Ltd., Tokyo 112-0002 (JP); Nakamura, Hirotoshi, Hamamatsu-shi, Shizuoka 431-3126 (JP)
(72) Inventor: NAKAMURA, Hirotoshi, Shizuoka 431-3126 (JP); KATO, Miki, Tokyo 112-0002 (JP); HIRAYAMA, Misaki, Tokyo 112-0002 (JP)
(74) Representative: Büchel, Edwin
(86) International application number: PCT/JP2024/039452
(87) International publication number: WO 2025/100444

(57) **Abstract**

A problem to be addressed by the present invention is to provide a new method for simply measuring the activity of thyroid-stimulating hormone (TSH). Provided is a reporter cell for measuring thyroid-stimulating hormone (TSH) activity, wherein a TSH receptor gene encoding a TSH receptor or an active fragment thereof and a reporter gene encoding a reporter protein are introduced into the reporter cell, and the reporter gene is placed under the control of a promoter whose promoter activity is regulated depending on the activity of the TSH receptor or the active fragment thereof.

## Description

### Technical Field

The present invention relates to a reporter cell for measuring the activity of thyroid-stimulating hormone (TSH), a kit for measuring TSH activity, a method for measuring TSH activity, a method for determining hypothyroidism or hyperthyroidism, and the like.

### Background Art

The amount of thyroid hormone in the blood is regulated by the hypothalamus and pituitary gland of the brain. Thyrotropin-releasing hormone (TRH) secreted from the hypothalamus stimulates the pituitary gland to promote the synthesis of thyroid-stimulating hormone (TSH). TSH secreted from the pituitary gland stimulates the thyroid gland to promote the production and secretion of thyroid hormones T4 and T3. Most of the thyroid hormones (T4 and T3) secreted into the blood exist bound to proteins in the blood, but a free thyroid hormone (mainly FT3) not bound to a protein enters a cell of a target tissue and binds to a receptor present in the cell nucleus to exert a hormonal effect. On the other hand, the free thyroid hormones are known to regulate the secretion of TSH from the pituitary gland by acting on the hypothalamus and the pituitary gland.

Graves' disease and Hashimoto's disease are well known as representative diseases causing abnormalities in thyroid function; the former results in hyperthyroidism due to an increase in free thyroid hormones in the blood, and the latter results in hypothyroidism due to a decrease in free thyroid hormones.

In addition, a pathological condition in which the blood TSH concentration is increased while the values of free thyroid hormones (FT4 and FT3) in the blood are within the normal range is called subclinical hypothyroidism, and conversely, a pathological condition in which the blood TSH concentration is decreased is called subclinical hyperthyroidism; these represent mild hypothyroidism and mild hyperthyroidism, respectively. The number of subjects falling under the condition of subclinical hypothyroidism (normal FT4 and FT3 concentrations, TSH concentration equal to or higher than the upper reference value of the normal range) is extremely large, and the Japan Endocrine Society states that 3.3 to 6.1% of "healthy" adults fall under this category.

Among patients with subclinical hypothyroidism, many complain of hypothyroid symptoms (fatigue, constipation, sensitivity to cold, dry skin, depressive mood, menstrual irregularity, and the like). It is known that there can be risks such as progression of arteriosclerosis, elevation of LDL cholesterol, cardiovascular disorders such as angina pectoris and myocardial infarction, non-alcoholic fatty liver disease (NAFLD), fatty liver, infertility, miscarriage/premature birth, and lowered fetal IQ.

As a treatment method for subclinical hypothyroidism, replacement therapy with a thyroid hormone drug such as levothyroxine is effective, similar to hypothyroidism. However, there is no internationally unified view on the level of TSH at which replacement therapy should be started. Generally, replacement therapy is recommended for a subject with a TSH concentration of 10 µIU/mL or higher.

For a subject with a TSH concentration of less than 10 µIU/mL, the decision to administer a thyroid hormone drug is difficult because comparing to the therapeutic benefit, the risk of a side effect due to overdose of a thyroid hormone drug can increase. Generally, replacement therapy is often applied to a subject with approximately 7 to 8 µIU/mL, but currently, individual judgments are made considering risk factors such as hyperlipidemia.

Regarding the efficacy of replacement therapy for subclinical hypothyroidism, a randomized double-blind placebo-controlled parallel-group comparison study (TRUST study) conducted in the past did not find efficacy in improving symptoms with levothyroxine for elderly patients. However, only small-scale randomized controlled trials have been conducted so far regarding levothyroxine replacement therapy for subclinical hypothyroidism. Accordingly, it cannot be said that sufficient evidence regarding the efficacy and risks of replacement therapy has been obtained so far (Non-Patent Literature 1 and 2).

On the other hand, thyroid hormone is an essential hormone extremely important for fetal growth and development. Since the fetus cannot yet produce sufficient thyroid hormone by itself, and since thyroid hormone in the maternal blood is the main source of supply, pregnant women are strongly required to avoid even a very mild hypothyroid state. Accordingly, it is internationally recommended for a pregnant woman to start thyroid hormone replacement therapy from a TSH level of 4 µIU/mL or higher, which is a high value within the normal range (or from a TSH level of 2.5 µIU/mL or higher if the pregnant woman is a Hashimoto's disease carrier).

### Citation List

### Non-Patent Literature

Non-Patent Literature 1: N Engl J Med. (2017) 376(26): 2534-2544.
Non-Patent Literature 2: JAMA. (2019) 322(20): 1977-1986.

### Summary of Invention

### Technical Problem

An object of the present invention is to provide a novel method for simply measuring the activity of thyroid-stimulating hormone (TSH).

### Solution to Problem

Currently, the most widely used method to quantify blood TSH is an immunological measurement method which based on antigen-antibody reaction. This method is based on sandwiching human TSH using two types of monoclonal antibodies capable of binding to TSH, and measures the amount of TSH. Currently, diagnosis and determination of treatment guidelines are conducted based on the amount of TSH measured by the immunological measurement method.

However, the immunological TSH measurement method merely measures the amount of TSH, and may not reflect the biological activity of TSH. The reason is that sugar chains are bound to TSH, and the structure thereof can vary depending on the individual, whereby biological activity such as thyroid-stimulating activity can be affected. Accordingly, even between subjects having the same amount of TSH measured by the immunological measurement method, the actual TSH activity can differ greatly. For example, it is possible that among subjects whose TSH concentration (TSH amount) measured by the immunological measurement method is approximately 7 to 8 µIU/mL, there are many subjects who actually have low TSH activity and hence require no replacement therapy, and that among subjects whose TSH concentration is less than approximately 7 to 8 µIU/mL, there are subjects who require replacement therapy. Accordingly, there is a possibility that the TSH amount measurement based on the conventional immunological measurement method does not accurately determine subclinical hypothyroidism.

In order to solve the above problems, the present inventors prepared a reporter cell for measuring TSH activity by introducing a TSH receptor gene and a luciferase gene that placed downstream of a TSH receptor which promoter is activated downstream . When this reporter cell is allowed to react with a serum sample comprising TSH, TSH binds to the TSH receptor on the cell surface, and luciferase is synthesized in the reporter cell depending on the degree of activation of the TSH receptor. The present inventors have discovered that TSH activity can be simply quantified by allowing luciferin to react with this luciferase and measuring the amount of luminescence using a luminometer.

The present inventors further used the above reporter cells to measure TSH activity in serum obtained from subclinical hypothyroidism. As a result, surprisingly, about 7% of subjects whose TSH concentration based on the conventional immunological measurement method was 10 µIU/mL or higher had an actual TSH activity of less than 7 µIU/mL and did not require replacement therapy. The present invention is based on the above research results and provides the following.

(1) A reporter cell for measuring thyroid-stimulating hormone (TSH) activity, wherein
   a TSH receptor gene encoding a TSH receptor or an active fragment thereof and a reporter gene encoding a reporter protein are introduced into the reporter cell, and
   the reporter gene is placed under the control of a promoter whose promoter activity is regulated depending on the activity of the TSH receptor or the active fragment thereof.
(2) The reporter cell of (1), wherein the promoter comprises a cAMP response element.
(3) The reporter cell of (1), wherein the reporter protein is a luminescent protein, a fluorescent protein, or an enzyme protein.
(4) The reporter cell of (1), wherein the reporter cell is a mammalian cell.
(5) The reporter cell of (4), wherein the mammalian cell is an HEK293 cell, a HeLa cell, an NIH3T3 cell, a CHO cell, a COS cell, or a Vero cell.
(6) A kit for measuring TSH activity, comprising the reporter cell of (1).
(7) The kit for measuring TSH activity of (6), further comprising a TSH standard solution containing a standard concentration of TSH.
(8) The kit for measuring TSH activity of (6) or (7), wherein
   the reporter protein is a luminescent protein, and
   the kit further comprises a luminescent substrate for the luminescent protein.
(9) A method for measuring the activity of thyroid-stimulating hormone (TSH) in a sample, comprising:
   a culturing step of culturing a reporter cell in a medium containing the sample; and
   a reporter activity measurement step of measuring the activity of the reporter protein derived from the reporter cell after the culturing, wherein
   a TSH receptor gene encoding a TSH receptor or an active fragment thereof and a reporter gene encoding a reporter protein are introduced into the reporter cell, and
   the reporter gene is placed under the control of a promoter whose promoter activity is regulated depending on the activity of the TSH receptor or the active fragment thereof.
(10) The method of (9), wherein
   the reporter protein is a luminescent protein, and
   the reporter activity measurement step comprises adding a luminescent substrate for the luminescent protein to the reporter cell and measuring the luminescence intensity of the luminescent substrate.
(11) A method for determining hypothyroidism or hyperthyroidism, comprising:
   a culturing step of culturing a reporter cell in a medium containing a body fluid derived from a subject;
   a reporter activity measurement step of measuring the activity of a reporter protein derived from the reporter cell after the culturing to produce a reporter activity value; and
   a determination step of determining that the subject has a overt hypothyroidism or an subclinical hypothyroidism when the reporter activity value or a TSH activity value calculated based thereon is higher than an upper reference value for TSH, and determining that the subject has a overt hyperthyroidism or an subclinical hyperthyroidism when the reporter activity value or the TSH activity value is lower than a lower reference value for TSH, wherein
   a TSH receptor gene encoding a TSH receptor or an active fragment thereof and a reporter gene encoding a reporter protein are introduced into the reporter cell, and
   the reporter gene is placed under the control of a promoter whose promoter activity is regulated depending on the activity of the TSH receptor or the active fragment thereof.
(12) A method for determining hypothyroidism or hyperthyroidism, comprising
   a culturing step of culturing a reporter cell in a medium containing a body fluid derived from a subject;
   a reporter activity measurement step of measuring the activity of a reporter protein derived from the reporter cell after the culturing to produce a reporter activity value;
   an FT4/FT3 measurement step of measuring the concentrations of free thyroxine (FT4) and free triiodothyronine (FT3) in the body fluid derived from the subject to produce an FT4 measurement value and an FT3 measurement value; and
   a determination step which comprises the following (a) to (d), based on the reporter activity value or a TSH activity value calculated based thereon, and the FT4 measurement value and the FT3 measurement value:
      (a) determining that the subject has a overt hypothyroidism when the reporter activity value or the TSH activity value is higher than the upper reference value for TSH and at least one of the FT4 measurement value and the FT3 measurement value is lower than a lower reference value thereof;
      (b) determining that the subject has an subclinical hypothyroidism when the reporter activity value or the TSH activity value is higher than the upper reference value for TSH and the FT4 measurement value and the FT3 measurement value are not lower than their respective lower reference values and not higher than their respective upper reference values;
      (c) determining that the subject has an subclinical hyperthyroidism when the reporter activity value or the TSH activity value is lower than a lower reference value for TSH and the FT4 and FT3 measurement values are not lower than their respective lower reference values and not higher than their respective upper reference values;
      (d) determining that the subject has a overt hyperthyroidism when the reporter activity value or the TSH activity value is lower than the lower reference value for TSH and at least one of the FT4 measurement value and FT3 measurement value is higher than an upper reference value thereof, wherein
   a TSH receptor gene encoding a TSH receptor or an active fragment thereof and a reporter gene encoding a reporter protein are introduced into the reporter cell, and
   the reporter gene is placed under the control of a promoter whose promoter activity is regulated depending on the activity of the TSH receptor or the active fragment thereof.
(13) The method of (11) or (12), wherein, in the determination step, the TSH activity value is calculated based on the reporter activity value and a standard reporter activity value which is produced based on a standard concentration of TSH.
(14) The method of (11) or (12), wherein
   the reporter protein is a luminescent protein, and
   the reporter activity measurement step comprises adding a luminescent substrate for the luminescent protein to the reporter cell and measuring the luminescence intensity of the luminescent substrate.
(15) The method of (11) or (12), wherein the body fluid is blood, serum, or plasma.
(16) The method of (11) or (12), wherein the lower reference value for TSH is 0.1 to 1.0 µIU/mL and/or the upper reference value for TSH is 4.0 to 9.0 µIU/mL.
(17) The method of (11) or (12), wherein the lower reference value for FT4 is 0.90 ng/dL, and the upper reference value for FT4 is 1.70 ng/dL, and/or the lower reference value for FT3 is 2.30 pg/mL, and the upper reference value for FT3 is 4.00 pg/mL.
(18) The method of (11) or (12), wherein the subject is pregnant or trying to conceive.
(19) The kit for measuring TSH activity of (6) or (7), for use in the method of (11) or (12).

The present specification encompasses the disclosure of Japanese Patent Application No. 2023-190104 that serves as the basis of the priority of the present application.

### Advantageous Effects of Invention

According to the reporter cell of the present invention, a new method for simply measuring the activity of thyroid-stimulating hormone (TSH) is provided. The present invention also provides a method for determining hypothyroidism or hyperthyroidism using the reporter cell of the present invention.

### Brief Description of Drawing

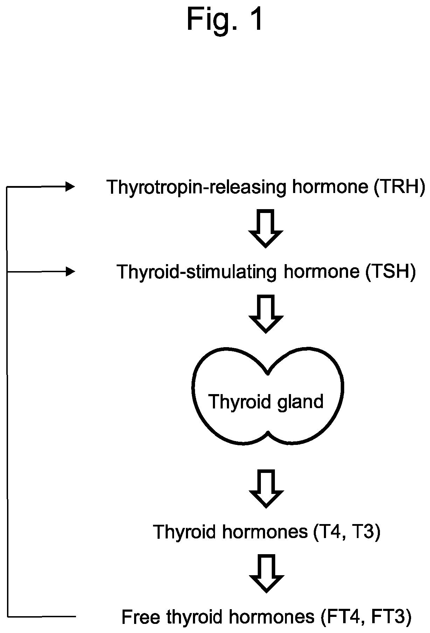

[Figure 1] Figure 1 schematically shows the regulation system of thyroid hormone.

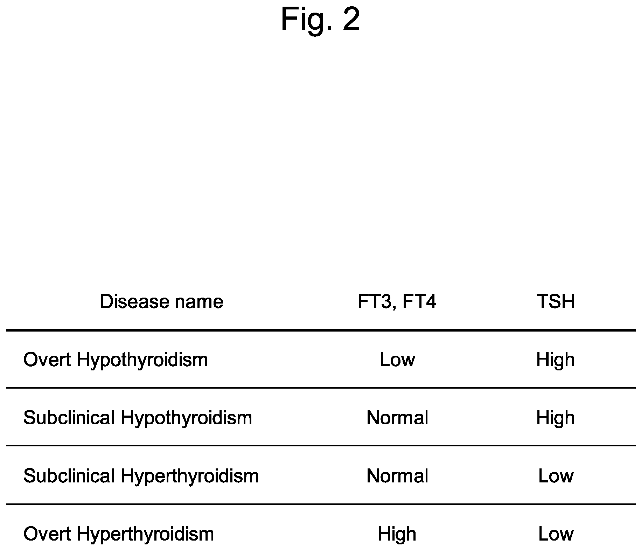

[Figure 2] Figure 2 shows the relationship between the following: overt hypothyroidism, subclinical hypothyroidism, subclinical hyperthyroidism, and overt hyperthyroidism; and blood thyroid-stimulating hormone (TSH) and free thyroid hormones (FT3 and FT4).

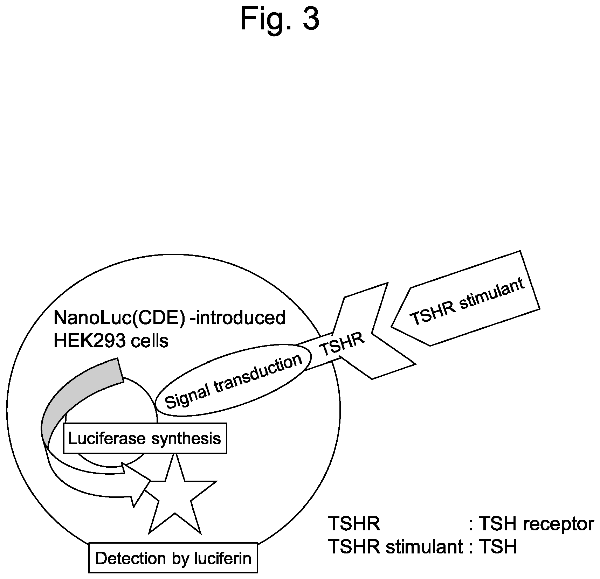

[Figure 3] Figure 3 schematically shows a reporter cell for measuring TSH activity. A TSH receptor gene and a luciferase gene placed downstream of a promoter activated downstream of the TSH receptor are introduced into the reporter cell.
[Figure 4] Figure 4 shows the results of quantifying the luminescence intensity of luciferase using TSH standard solutions. Figure 4A shows the TSH concentration (µIU/mL) in each TSH standard solution, the luminescence intensity (RLU) exhibited by the reporter cells for each TSH standard solution, and the relative luminescence intensity (%) when the luminescence intensity of 0µIU/mL is 100%. Figure 4B shows a standard curve graphing the relationship between TSH concentration and relative luminescence intensity.
[Figure 5] Figure 5 shows the results of measuring the amount of TSH in serum obtained from human subjects (n = 284) by an immunological measurement method based on an antigen-antibody reaction (horizontal axis), and the results of measuring reporter activity using reporter cells (vertical axis).
[Figure 6] Figure 6 shows the results of measuring the activity of TSH in serum obtained from healthy subjects (n = 267), patients with subclinical hypothyroidism (n = 89), and patients with overt hypothyroidism (n = 121) by a reporter activity measurement method based on reporter cells.

### Description of Embodiments

### 1. Reporter cell

### 1-1. Overview

A first aspect of the present invention is a reporter cell for measuring the activity of thyroid-stimulating hormone (TSH). The reporter cell of the present invention has a TSH receptor gene and a reporter gene introduced thereinto. In the reporter cell of the present invention, the reporter gene is placed under the control of a promoter whose promoter activity is regulated depending on the activity of the TSH receptor or an active fragment thereof. Accordingly, when TSH binds to the TSH receptor on the reporter cell, the expression level of the reporter gene increases. Because of this, the activity of TSH can be measured by measuring the activity of a reporter protein encoded by the reporter gene.

### 1-2. Definitions

The following terms frequently used in this specification are defined.

"Thyroid-stimulating hormone" (often referred to as "TSH" in this specification) is a glycoprotein hormone consisting of an α subunit and a β subunit. TSH is secreted from the pituitary gland and stimulates the thyroid gland to promote the secretion of thyroid hormones T4 and T3. In thyroid dysfunction accompanied by an abnormality in blood TSH concentration, a pathological condition in which the TSH concentration increases is called (overt or subclinical) hypothyroidism, and a pathological condition in which the TSH concentration decreases is called (overt or subclinical) hyperthyroidism. In this specification, the biological species of origin of TSH is not particularly limited, and the biological species of origin of the α subunit and β subunit constituting TSH are also not limited. Specific examples of the α subunit of TSH include a human-derived α subunit consisting of the amino acid sequence of SEQ ID NO: 1. Specific examples of the β subunit of TSH include a human-derived β subunit consisting of the amino acid sequence of SEQ ID NO: 2.

In this specification, "thyroid-stimulating hormone receptor" (TSHR, often referred to as "TSH receptor" in this specification) refers to a G protein-coupled receptor capable of binding to TSH as a receptor for TSH. TSH receptors are known to be expressed in epithelial cells of the thyroid gland. In this specification, the biological species of origin of the TSH receptor is not particularly limited. Examples of the TSH receptor include a human-derived TSH receptor, a TSH receptor ortholog derived from a biological species other than human, and a mutant of the TSH receptor or TSH receptor ortholog. Specific examples of the human-derived TSH receptor include a human-derived TSH receptor consisting of the amino acid sequence of SEQ ID NO: 3. Examples of mutants or orthologs of the TSH receptor include amino acid sequences in which one or several (for example, 2 to 10, 2 to 7, 2 to 5, 2 to 4, or 2 to 3) amino acids are deleted, substituted, and/or added in the amino acid sequence shown in SEQ ID NO: 3, and amino acid sequences having 60% or more (for example, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more) amino acid identity with the amino acid sequence shown in SEQ ID NO: 3.

In this specification, "amino acid identity" refers to the ratio (%) of identical amino acid residues between two amino acid sequences relative to the total amino acid residues when the two amino acid sequences are aligned, introducing gaps as necessary, so that the degree of amino acid matching between the two is maximized. Amino acid identity can be calculated using a protein search system such as BLAST or FASTA.

In this specification, an "active fragment" of a TSH receptor refers to any fragment capable of regulating the transcriptional activity of a reporter gene depending on binding to TSH in the reporter cell of the present invention. For example, the active fragment may be a polypeptide fragment retaining 20% or more, 30% or more, 40% or more, 50% or more, 60% or more, 70% or more, 80% or more, 90% or more, 95% or more, 98% or more, or equivalent or more activity of the full-length TSH receptor. The amino acid length of the active fragment is not particularly limited as long as the fragment retains the activity of the TSH receptor. For example, the amino acid length may be 500 or more, 600 or more, or 700 or more consecutive amino acid residues comprising a transmembrane domain in the full-length amino acid sequence of the TSH receptor, and may be a polypeptide consisting of 750 or less, 700 or less, or 600 or less consecutive amino acid residues.

In this specification, "TSH receptor gene" is a gene encoding a TSH receptor. Examples of the TSH receptor gene include a gene encoding a human-derived TSH receptor, a TSH receptor ortholog derived from a biological species other than human, or a mutant of a TSH receptor or TSH receptor ortholog. Specific examples of the gene encoding a human-derived TSH receptor include a gene encoding a human-derived TSH receptor consisting of the amino acid sequence of SEQ ID NO: 3, for example, a human TSH receptor gene consisting of the base sequence of SEQ ID NO: 4. Examples of the gene encoding a mutant or ortholog of the TSH receptor include a base sequence having 60% or more (for example, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more) base identity with the base sequence of SEQ ID NO: 4.

In this specification, "base identity" refers to the ratio (%) of identical bases between two base sequences relative to the total base sequence when the two base sequences are aligned, introducing gaps as necessary, so that the degree of base matching between the two is maximized.

In this specification, "reporter cell" refers to a cell capable of exhibiting a change in the below-described reporter activity depending on the presence or amount of extracellular TSH, unless otherwise specified.

In this specification, "reporter activity" refers to an activity enabling determination of the presence or absence of TSH and/or measurement of the amount of TSH, unless otherwise specified. More specifically, reporter activity is the activity of the below-described reporter protein expressed in the reporter cell. The type of reporter activity is not particularly limited, but specific examples include luminescent activity of a luminescent protein, fluorescent activity of a fluorescent protein, and enzymatic activity of an enzyme protein.

In this specification, "reporter protein" refers to a polypeptide or peptide expressed by a reporter cell and enabling determination of the presence or absence of extracellular TSH or measurement of the amount of extracellular TSH. The type of reporter protein is not particularly limited, but specific examples thereof include a luminescent protein, a fluorescent protein, and an enzyme protein (for example, a chromogenic protein).

"Luminescent protein" refers to a substrate protein capable of emitting light without requiring excitation light, or an enzyme catalyzing the light emission of the substrate protein. Examples include luciferin or aequorin as substrate proteins, and luciferase (for example, Renilla luciferase, Gaussia luciferase, firefly luciferase, and bacterial luciferase) as enzymes.

"Fluorescent protein" refers to a protein that emits fluorescence of a specific wavelength when irradiated with excitation light of a specific wavelength. The fluorescent protein may be either natural or non-natural. In addition, the excitation wavelength and fluorescence wavelength are not particularly limited. Specifically, examples include CFP, BFP, RFP, mCherry, DsRed (including derivatives such as 3xP3-DsRed), YFP, PE, PerCP, APC, GFP (including derivatives such as EGFP, 3xP3-EGFP), and the like.

"Chromogenic protein" is a protein involved in the biosynthesis of a pigment, or a protein enabling chemical detection by a pigment upon provision of a substrate, and is usually an enzyme. Specific examples of such enzymes include horseradish peroxidase (HRP), alkaline phosphatase (AP), β-galactosidase (LacZ), β-glucuronidase (GUS), melanin pigment synthesis proteins, and the like.

As the reporter protein, in addition to the above examples, tag peptides or epitope sequences detectable by antibodies or the like can also be used.

In this specification, "reporter gene" is a gene encoding a reporter protein. Examples of the reporter gene include a luminescent protein gene, a fluorescent protein gene, and an enzyme gene (for example, a chromogenic protein gene).

### 1-3. Constitution

In the reporter cell for measuring the activity of thyroid-stimulating hormone (TSH) of this aspect, a TSH receptor gene encoding a TSH receptor or an active fragment thereof and a reporter gene encoding a reporter protein are introduced. In the reporter cell of this aspect, the reporter gene is placed under the control of a promoter whose promoter activity is regulated depending on the activity of the TSH receptor or the active fragment thereof.

The type of reporter cell of this aspect is not limited. The reporter cell may be either a prokaryotic cell or a eukaryotic cell. Examples of prokaryotic cells include bacterial cells such as *E*. *coli* cells. Examples of eukaryotic cells include fungal cells (for example, yeast cells), algal cells, plant cells, protozoan cells, insect cells, nematode cells, fish cells, avian cells (for example, chicken cells), and mammalian cells (for example, mouse cells, chimpanzee cells, and human cells). The reporter cell is preferably a mammalian cell. Specific examples of mammalian cells include, but are not limited to, HEK293 cells, HeLa cells, NIH3T3 cells, CHO cells, COS cells, or Vero cells. The host cell may be either an adherent cell or a suspension cell, but is more preferably an adherent cell.

In this specification, "a promoter whose promoter activity is regulated depending on the activity of the TSH receptor or the active fragment thereof" refers to any promoter whose transcriptional activity of a gene placed in the promoter downstream region changes (for example, activated or inactivated) in response to binding of TSH to the TSH receptor or the active fragment thereof expressed by the reporter cell, and the specific type is not limited. Examples of such a promoter include a promoter comprising a cAMP response element. For example, a promoter in which one or more cAMP response elements are combined with a minimal Herpes Simplex Virus (HSV) thymidine kinase gene promoter can be used.

In this specification, "cAMP response element (CRE)" means a base sequence that regulates (for example, promotes or suppresses) transcriptional activity of a gene in response to an increase in intracellular cAMP concentration. Various base sequences are known as cAMP response elements, and any base sequence may be used as long as the base sequence is a cAMP response element known in the art. Specific examples of the cAMP response element include the base sequence TGACGTCA (SEQ ID NO: 5), and sequences in which one or several (for example, 2 to 3) bases are deleted, substituted, and/or added in the base sequence TGACGTCA (SEQ ID NO: 5) and which have cAMP response activity.

In this specification, the reporter gene being "placed under the control of a promoter" means that the reporter gene is placed in a state where the gene can be expressed in the promoter downstream region under the control of the promoter.

In this specification, the TSH receptor gene and the reporter gene being "introduced" into the reporter cell means that the TSH receptor gene and the reporter gene are comprised in a gene expression vector introduced into the reporter cell and/or integrated into the genome of the reporter cell. For example, both the TSH receptor gene and the reporter gene may be comprised in the same gene expression vector, or the TSH receptor gene and the reporter gene may be comprised in two or more different gene expression vectors.

In this specification, "gene expression vector" refers to a vector comprising a target gene such as a reporter gene or a TSH receptor gene in an expressible state, and encompassing an expression unit capable of controlling the expression of the target gene. The gene expression vector may be a plasmid vector or a viral vector. The plasmid vector may be a commercially available expression vector for mammalian cells such as a pSI vector from Promega Corporation, or a shuttle vector capable of replicating between a mammalian cell and a bacterium such as *E. coli*. As the viral vector, viral vectors such as retroviral vectors (including oncoretroviral vectors, lentiviral vectors, and pseudotyped vectors), adenoviral vectors, adeno-associated virus (AAV) vectors, simian virus vectors, vaccinia virus vectors, Sendai virus vectors, Epstein-Barr virus (EBV) vectors, and HSV vectors can be used. The gene expression vector may comprise, in addition to the target gene and the promoter, a component such as a marker gene, intron, enhancer, terminator, origin of replication, and/or poly-A signal, if necessary.

The reporter cell of this aspect may comprise, in addition to the above-mentioned TSH receptor gene and reporter gene, a gene encoding a signal transduction factor that regulates promoter activity depending on the activity of the TSH receptor or an active fragment thereof. For example, as a G protein that can be activated by the TSH receptor or an active fragment thereof, a gene encoding a Gαₛ protein (for example, human GNAS gene consisting of the base sequence of SEQ ID NO: 5), an adenylate cyclase gene, a protein kinase A gene (PKA gene), and/or a cAMP response element binding protein gene (CREB gene) may be introduced into the reporter cell.

### 1-4. Effects

In the reporter cell of the present invention, the transcription level of the reporter gene is regulated depending on the presence or amount of extracellular TSH. For example, the higher the presence or amount of extracellular TSH, the more the transcription amount of the reporter gene increases, or the lower the presence or amount of extracellular TSH, the more the transcription amount of the reporter gene decreases. Accordingly, by evaluating TSH activity through measuring reporter activity based on the reporter protein encoded by the reporter gene, it becomes possible to determine the presence of TSH and measure the amount of TSH.

### 2. Kit for measuring TSH activity

### 2-1. Overview

A second aspect of the present invention is a kit for measuring TSH activity. The kit for measuring TSH activity of this aspect comprises the reporter cell of the first aspect. According to the kit for measuring TSH activity of this aspect, TSH activity can be evaluated by quantifying the presence or amount of TSH in an arbitrary sample as reporter activity. The kit for measuring TSH activity of this aspect can also be used in the method of the below-described third aspect or fourth aspect.

### 2-2. Constitution

The kit for measuring TSH activity of this aspect comprises the reporter cell of the first aspect. The constitution of the reporter cell is as described in the first aspect. Accordingly, specific description thereof is omitted here.

In one embodiment, the kit for measuring TSH activity of this aspect further comprises a TSH standard solution in addition to the reporter cell. In this specification, "TSH standard solution" refers to a liquid (for example, aqueous solution) containing a standard concentration of TSH. The standard concentration of TSH is not particularly limited, and may be, for example, 0 µIU/mL to 1000 µIU/mL, 1 µIU/mL to 500 µIU/mL, 5 µIU/mL to 200 µIU/mL, 10 µIU/mL to 100 µIU/mL, or 20 µIU/mL to 50 µIU/mL. By bringing a TSH standard solution containing a standard concentration of TSH into contact with the reporter cell of the present invention and measuring a reference reporter activity, it becomes possible to evaluate the presence or amount of TSH in a sample on the basis of the reporter activity obtained for the measurement target sample.

In addition, the kit for measuring TSH activity of this aspect may comprise a substrate for the reporter protein. The substrate for the reporter protein may be suitably selected according to the type of the reporter protein. For example, if the reporter protein is a luminescent protein such as luciferase, the substrate may be a luminescent substrate such as luciferin. If the reporter protein is a chromogenic protein, the substrate may be a chromogenic substrate.

The kit for measuring TSH activity of this aspect may also comprise a medium for culturing the reporter cell, a drug such as an antibiotic for addition to the medium, and/or instructions for use.

### 3. Method for measuring TSH activity

### 3-1. Overview

A third aspect of the present invention is a method for measuring the activity of thyroid-stimulating hormone (TSH) in a sample (hereinafter referred to as "TSH activity measurement method"). According to the measurement method of the present invention, the presence or amount of TSH in a sample can be accurately and simply measured.

### 3-2. Method

The TSH activity measurement method of this aspect comprises a culturing step and a reporter activity measurement step as essential steps, and a TSH standard solution measurement step as an optional step. Each step will be specifically described below.

### (1) Culturing step

The "culturing step" is a step of culturing the reporter cell in a medium containing the sample. The specific constitution of the reporter cell conforms to the description in the first aspect.

In the present specification, the "sample" is not particularly limited as long as the sample comprises or can comprise TSH, but usually corresponds to a liquid sample. Examples of the liquid sample include a liquid isolated from a living body, such as an isolated body fluid derived from a subject. The sample can be diluted as necessary, such as when reporter activity exceeds a measurable range in the below-described reporter activity measurement step.

Examples of the "body fluid" in the present specification include blood (including serum, plasma, and interstitial fluid), lymph, cerebrospinal fluid, interstitial fluid, pleural effusion, sputum, tears, nasal discharge, saliva, urine, ascites, extracts of tissues or cells, and the like. The blood may be serum or plasma prepared from blood. For example, blood, serum, plasma, cerebrospinal fluid, urine, saliva, and ascites are preferable because collection thereof is relatively easy. The body fluid may be derived from a healthy subject or a patient with an arbitrary disease. For example, the body fluid may be derived from a patient with thyroid dysfunction or a patient suspected of having thyroid dysfunction. The serum may be used after being inactivated.

The type of the medium used in this step is not limited as long as the medium does not inhibit binding between TSH and the TSH receptor and can maintain the reporter cell as a living cell. For example, the medium may be a medium known in the art and generally used in cell culture. The medium may be any of a basal medium, a serum-free medium, a low-serum medium, and a serum-supplemented medium, and may usually be a basal medium, for example, a standard cell culture medium. The "standard cell culture medium" herein refers to a highly versatile basal medium mainly used for culturing various types of cells derived from mammals. Specifically, examples thereof include Eagle MEM (Eagle Minimum Essential Medium), DMEM (Dulbecco's Modified Eagle Medium), Ham F10 (Ham's Nutrient Mixture F10) medium, Ham F12 (Ham's Nutrient Mixture F12) medium, M199 medium, High Performance Medium 199, RPMI-1640 (Roswell Park Memorial Institute-1640) medium, and DMEM/F12 (Dulbecco's Modified Eagle Medium/Ham's Nutrient Mixture F12) medium. Specific compositions of the standard cell culture medium are known in the art, and accordingly may be prepared based on compositions described in suitable literature (for example, Kaech S. and Banker G., 2006, Nat. PROtoc., 1(5): 2406-2415).

As culture conditions in this step, conditions capable of inducing expression of the reporter gene can be used under culture conditions known in the art depending on the type and origin of the reporter cell. Usually, culturing may be performed at 5% CO₂ and 30°C to 40°C, preferably 35°C to 38°C (for example, 37°C).

The culture time of this step varies depending on, for example, the types of the reporter cell and the reporter protein, but is 1 hour to 72 hours, 2 hours to 36 hours, or 3 hours to 24 hours, and preferably 2 hours to 6 hours, in order to achieve the above object of this step.

### (2) Reporter activity measurement step

The "reporter activity measurement step" is a step of measuring reporter activity based on the reporter protein. In this step, TSH activity can be quantitatively analyzed by detecting and quantifying the reporter activity.

The measurement method of this step varies depending on the type of reporter activity comprised in the reporter protein, but any method can be easily determined by a person skilled in the art. When the reporter protein is a luminescent protein, luminescent activity in the reporter cell and/or in the culture supernatant can be measured by applying a luminescent substrate (for example, luciferin) for the luminescent protein. When the reporter protein is a fluorescent protein, fluorescent activity comprised in the reporter cell and/or in the culture supernatant can be measured based on an excitation wavelength and a fluorescence wavelength depending on the type of the fluorescent protein. When the reporter protein is an enzyme such as HRP, enzymatic activity in the reporter cell and/or in the culture supernatant can be measured by applying a chromogenic substrate (for example, an HRP substrate such as TMB, 4-CN, or DAB; an AP substrate such as BCIP/NBT; and a LacZ substrate such as X-Gal).

### (3) TSH standard solution measurement step

The "TSH standard solution measurement step" is a step of obtaining a measurement value of reporter activity for a standard concentration of TSH by performing the culturing step and the reporter activity measurement step using a TSH standard solution containing the standard concentration of TSH as a sample. By comparing the measurement value of the reporter activity for the standard concentration of TSH obtained in this step with the measurement value obtained for a target sample in the reporter activity measurement step of (2) above, presence of TSH in the target sample can be determined, and the amount of TSH can be quantified. For example, by measuring reporter activities for a plurality of standard concentrations of TSH in this step to create a standard curve, the presence of TSH in the target sample can be determined, and the amount or concentration of TSH can be estimated from the measurement value obtained for the target sample in the reporter activity measurement step of (2) above.

### 3-3. Effects

According to the TSH activity measurement method of this aspect, TSH activity in a sample can be evaluated with extremely high sensitivity and reliability. For example, compared to the conventional ELISA method, the sensitivity and/or accuracy of the TSH activity measurement method of this aspect is overwhelmingly higher.

### 4. Method for determining hypothyroidism or hyperthyroidism

### 4-1. Overview

A fourth aspect of the present invention is a method for determining hypothyroidism or hyperthyroidism. According to the determination method of the present invention, hypothyroidism or hyperthyroidism of a subject can be determined by evaluating the activity of TSH in a body fluid derived from the subject.

### 4-2. Definitions

In the present specification, "hypothyroidism" refers to a state in which the synthesis amount of thyroid hormone is lower than a normal value. Among cases of hypothyroidism, a case where a thyroid hormone measurement value is lower than a normal range and a TSH measurement value is higher than a normal range as clinical laboratory values is referred to as "overt hypothyroidism", and a case where a thyroid hormone measurement value is within a normal range and a TSH measurement value is higher than a normal range is referred to as "subclinical hypothyroidism". It is known that in a patient with subclinical hypothyroidism, there are risks such as progression of arteriosclerosis, elevation of LDL cholesterol, cardiovascular disorders such as angina pectoris and myocardial infarction, non-alcoholic fatty liver disease (NAFLD), fatty liver, infertility, miscarriage/premature birth, and lowered fetal IQ, there are risks such as the same symptoms as overt hypothyroidism (fatigue, constipation, sensitivity to cold, dry skin, depressive mood, menstrual irregularity, or the like).

In the present specification, "hyperthyroidism" refers to a state in which the synthesis amount of thyroid hormone is higher than a normal value. Among cases of hyperthyroidism, a case where a thyroid hormone measurement value is higher than a normal range and a TSH measurement value is lower than a normal range as clinical laboratory values is referred to as "overt hyperthyroidism", and a case where a thyroid hormone measurement value is within a normal range and a TSH measurement value is lower than a normal range is referred to as "subclinical hyperthyroidism". Examples of overt hyperthyroidism include Graves' disease, painless thyroiditis, subacute thyroiditis, and thyroid nodules.

In the present specification, "subject" is a living body to be an application target of the determination method of this aspect. Examples thereof include humans (in this case, particularly referred to as a "human subject"), livestock animals (cows, horses, sheep, goats, pigs, and the like), racehorses, pet animals (dogs, cats, rabbits, and the like), laboratory animals (mice, rats, guinea pigs, monkeys, marmosets, and the like), and wild/zoo animals (chimpanzees, wildcats, squirrels, and the like). Examples thereof include living bodies such as humans or mammals having or having a possibility of developing thyroid dysfunction such as hypothyroidism or hyperthyroidism. The subject may be, for example, a pregnant woman or a person trying to conceive. In addition, the subject may be a patient with hypothyroidism after surgery for thyroid cancer (for example, total or hemithyroidectomy), a patient with papillary thyroid microcarcinoma, or a case in which a TSH measurement value obtained by a conventional immunological measurement method is inconsistent with trends of an FT4 measurement value and an FT3 measurement value (for example, a case having macro TSH, anti-T4 antibody, anti-T3 antibody, and the like).

In the present specification, "healthy subject" refers to an individual not suffering from a specific disease such as thyroid dysfunction, preferably an individual not suffering from any disease, unless otherwise specified.

In the present specification, "control" corresponds to a healthy subject, and corresponds to an individual not suffering from a specific disease such as thyroid dysfunction, preferably an individual not suffering from any disease. The control may be a single individual or a plurality of individuals.

The term "determination" used in the present specification can be rephrased as determination support or assistance in determination.

### 4-3. Method

The determination method of this aspect comprises a culturing step, a reporter activity measurement step, and a determination step as essential steps, and comprises an FT4/FT3 measurement step and/or a TSH standard solution measurement step as optional steps. Each step will be specifically described below.

### (1) Culturing step

The "culturing step" is a step of culturing a reporter cell in a medium containing a body fluid derived from a subject. A specific constitution of this step conforms to the description of the culturing step in the third aspect.

### (2) Reporter activity measurement step

The "reporter activity measurement step" is a step of measuring the activity of a reporter protein derived from the reporter cell after the culturing in the culturing step to obtain a reporter activity value. A specific constitution of this step conforms to the description of the reporter activity measurement step in the third aspect.

### (3) FT4/FT3 measurement step

The "FT4/FT3 measurement step" is a step of measuring concentrations of free thyroxine (FT4) and/or free triiodothyronine (FT3) in the body fluid derived from the subject. The concentrations of FT4 and FT3 can be measured by a method known in the art. For example, an immunological measurement method such as ELISA or RIA (radioimmunoassay) using antibodies against FT4 and FT3, or a mass spectrometry method such as LC-MS/MS can be used. By this step, an FT4 measurement value and an FT3 measurement value are obtained.

### (4) Determination step

In one embodiment of this aspect, the "determination step" is a step of determining that the subject has a overt hypothyroidism or an subclinical hypothyroidism when the reporter activity value or a TSH activity value calculated based thereon is higher than an upper reference value for TSH, and determining that the subject has a overt hyperthyroidism or an subclinical hyperthyroidism when the reporter activity value or the TSH activity value is lower than a lower reference value for TSH.

In another embodiment of this aspect, the "determination step" comprises the following (a) to (d), based on the reporter activity value or a TSH activity value calculated based thereon, and the FT4 measurement value and the FT3 measurement value:
(a) determining that the subject has a overt hypothyroidism when the reporter activity value or the TSH activity value is higher than the upper reference value for TSH and at least one of the FT4 measurement value and the FT3 measurement value is lower than a lower reference value thereof;
(b) determining that the subject has an subclinical hypothyroidism when the reporter activity value or the TSH activity value is higher than the upper reference value for TSH and the FT4 measurement value and the FT3 measurement value fall within their respective lower and upper reference values;
(c) determining that the subject has an subclinical hyperthyroidism when the reporter activity value or the TSH activity value is lower than a lower reference value for TSH and the FT4 and FT3 measurement values fall within their respective lower and upper reference values;
(d) determining that the subject has a overt hyperthyroidism when the reporter activity value or the TSH activity value is lower than the lower reference value for TSH and at least one of the FT4 measurement value and FT3 measurement value is higher than an upper reference value thereof.

In the determination step of any of the above embodiments, the upper reference value for TSH (upper reference value for TSH concentration or TSH activity value) and the lower reference value for TSH (lower reference value for TSH concentration or TSH activity value) refer to boundary values for determining hypothyroidism and hyperthyroidism of the subject, respectively. For example, the upper reference value and the lower reference value for TSH may be an upper limit value and a lower limit value of a normal range regarding TSH concentration or TSH activity value, respectively, or may be numerical values based on body fluids of patients with hypothyroidism and/or hyperthyroidism and/or healthy subjects.

For example, the upper reference value for TSH concentration or TSH activity value may be 4.0 µIU/mL to 12.0 µIU/mL, 5.0 µIU/mL to 10.0 µIU/mL, 6.0 µIU/mL to 9.0 µIU/mL, or 7.0 µIU/mL to 8.0 µIU/mL, for example, 7.0 µIU/mL or 8.0 µIU/mL. The lower reference value for TSH concentration or TSH activity value may be 0.1 µIU/mL to 2.0 µIU/mL, 0.2 µIU/mL to 1.5 µIU/mL, 0.3 µIU/mL to 1.0 µIU/mL, 0.4 µIU/mL to 0.8 µIU/mL, or 0.5 µIU/mL to 0.6 µIU/mL, for example, 0.5 µIU/mL or 0.6 µIU/mL. Alternatively, based on results of the below-described Example 4, the upper reference value for TSH concentration or TSH activity value may be 4.0 µIU/mL to 10.0 µIU/mL, 5.0 µIU/mL to 9.0 µIU/mL, or 6.0 µIU/mL to 8.0 µIU/mL, for example, 7.0 µU/mL or 8.0 µIU/mL, and the lower reference value for TSH concentration or TSH activity value may be 0.01 µIU/mL to 2.0 µIU/mL, 0.05 µIU/mL to 1.5 µIU/mL, or 0.1 µIU/mL to 1.0 µIU/mL, for example, 0.1 µIU/mL or 1 µIU/mL. Note that in the present specification, TSH concentration corresponding to the TSH amount in a predetermined volume and TSH activity value indicating biological activity of TSH in a predetermined volume are both expressed using the unit "µIU/mL". The TSH concentration or TSH activity value in µIU/mL units can be determined by, for example, purchasing a TSH preparation from the National Institute for Biological Standards and Control (NIBSC), measuring a reference measurement value, and comparing the values.

In addition, as numerical values based on body fluids of controls such as healthy subjects, the upper reference value and the lower reference value can be defined based on a normal range corresponding to TSH activity values (range) measured for body fluids derived from controls. Alternatively, the values may be calculated based on sensitivity and specificity calculated from prevalence of hyperthyroidism and hypothyroidism and an ROC curve (receiver operating characteristic curve).

Furthermore, a TSH activity value or an average value thereof of a healthy subject not suffering from hyperthyroidism or hypothyroidism, or 1.5 times or more, 2 times or more, 3 times or more, 4 times or more, 5 times or more, or 6 times or more of any of these values can be set as the upper reference value regarding TSH concentration or TSH activity value. In addition, a TSH activity value or an average value thereof of a healthy subject not suffering from hyperthyroidism or hypothyroidism, or 0.8 times or less, 0.5 times or less, 0.4 times or less, 0.3 times or less, 0.2 times or less, or 0.1 times or less of any of these values can be set as the lower reference value regarding TSH concentration or TSH activity value.

In addition, measurement values obtained from a control group can be classified by percentile, and percentile values used for the classification can be used as the upper reference value and the lower reference value regarding TSH concentration or TSH activity value. For example, the 95th percentile of TSH activity values obtained from controls is set as the upper reference value, and if the TSH activity value of the subject is the 95th percentile or more, it can be determined that the subject has hypothyroidism (overt hypothyroidism or subclinical hypothyroidism). Similarly, the 5th percentile of TSH activity values obtained from controls is set as the lower reference value, and if the TSH activity value of the subject is the 5th percentile or less, it can be determined that the subject has hyperthyroidism (overt hyperthyroidism or subclinical hyperthyroidism).

Note that the TSH activity value of the healthy subject used as a control does not necessarily need to be measured each time, unlike the TSH activity value of the subject. For example, if the amount of sample used for measurement, measurement method, and measurement conditions are kept constant, TSH activity values of control healthy subjects measured previously can be reused.

In one embodiment, the TSH activity value in the determination step can be calculated based on the reporter activity value obtained in the reporter activity measurement step and a standard reporter activity value obtained for a TSH standard solution with a standard concentration.

In the determination step of any of the above, the upper reference values for the FT4 measurement value and the FT3 measurement value are boundary values for determining subclinical hyperthyroidism or overt hyperthyroidism of the subject, and the lower reference values for the FT4 measurement value and the FT3 measurement value are boundary values for determining subclinical hypothyroidism or overt hypothyroidism of the subject. For example, the upper reference values and the lower reference values for the FT4 measurement value and the FT3 measurement value may be an upper limit value and a lower limit value of a normal range regarding the FT4 measurement value and the FT3 measurement value, respectively, or may be numerical values based on measurement of controls such as healthy subjects.

For example, the upper reference value for the FT4 measurement value may be 1 ng/dL to 3 ng/dL, 1.25 ng/dL to 2 ng/dL, or 1.5 ng/dL to 1.8 ng/dL, for example, 1.7 ng/dL. In addition, the lower reference value for the FT4 measurement value may be 0.3 ng/dL to 1.5 ng/dL, 0.5 ng/dL to 1.2 ng/dL, or 0.7 ng/dL to 1.0 ng/dL, for example, 0.9 ng/dL. The upper reference value for the FT3 measurement value may be 2.5 pg/mL to 6.0 pg/mL, 3.0 pg/mL to 5.0 pg/mL, or 3.5 pg/mL to 4.5 pg/mL, for example, 4.0 pg/mL. In addition, the lower reference value for the FT3 measurement value may be 1.0 pg/mL to 3.5 pg/mL, 1.5 pg/mL to 3.0 pg/mL, or 2.0 pg/mL to 2.5 pg/mL, for example, 2.3 pg/mL.

As numerical values based on measurement of controls, the upper reference value and the lower reference value corresponding to normal ranges of FT4 measurement values and FT3 measurement values can be defined based on FT4 measurement values (ranges) and FT3 measurement values (ranges) measured for body fluids derived from controls. For example, the values can be calculated based on sensitivity and specificity calculated from prevalence of overt or subclinical hyperthyroidism, or overt or subclinical hypothyroidism, and an ROC curve (receiver operating characteristic curve).

Alternatively, similar to the above-mentioned method, an FT4 measurement value and an FT3 measurement value or an average value thereof of a healthy subject, or 1.5 times or more, 2 times or more, 3 times or more, 4 times or more, 5 times or more, or 6 times or more of any of these values can be set as the upper reference values for the FT4 measurement value and the FT3 measurement value. In addition, an FT4 measurement value and an FT3 measurement value or an average value thereof of a healthy subject, or 0.8 times or less, 0.5 times or less, 0.4 times or less, 0.3 times or less, 0.2 times or less, or 0.1 times or less of any of these values can be set as the lower reference values for the FT4 measurement value and the FT3 measurement value.

Furthermore, measurement values obtained from a control group can be classified by percentile, and percentile values used for the classification can be used as the upper reference values and the lower reference values for the FT4 measurement value and the FT3 measurement value.

The determination method of the present invention can improve accuracy of determination by being combined with other determination methods (for example, diagnosis regarding symptoms of thyroid dysfunction, medical interview by a doctor, genetic diagnosis, and the like).

Based on a determination result of the determination method of the present invention, replacement therapy with a thyroid hormone drug can be performed, and a treatment policy for thyroid dysfunction (for example, the type, dosage, administration interval, and the like of a thyroid hormone drug such as levothyroxine) can be determined.

Based on the measurement method or determination method of the present invention, replacement therapy with a thyroid hormone drug can be suitably started for, for example, a patient with subclinical hypothyroidism (for example, a person trying to conceive or a pregnant woman). In addition, when a causative disease such as Graves' disease or a functional nodule is not detected in a patient with subclinical hyperthyroidism diagnosed based on a TSH measurement value obtained by an immunological measurement method, subclinical hyperthyroidism can be accurately diagnosed by the method of the present invention.

In addition, in a patient with hypothyroidism after surgery for thyroid cancer (for example, total or hemithyroidectomy), replacement therapy with a thyroid hormone drug such as LT4 may be applied for the purpose of preventing recurrence of thyroid cancer, and it can be accurately monitored by the method of the present invention that TSH activity is controlled to a desirable level. Furthermore, regarding papillary thyroid microcarcinoma, a response method of follow-up observation without immediately performing surgery may be adopted, and in this case, it is required that the TSH activity value is not high. By suitably measuring TSH activity by the method of the present invention, it becomes possible to suitably determine whether to perform surgery for papillary thyroid microcarcinoma or to perform follow-up observation.

Furthermore, in a case where a TSH measurement value obtained by a conventional immunological measurement method is inconsistent with trends of an FT4 measurement value and an FT3 measurement value (a case having macro TSH, anti-T4 antibody, anti-T3 antibody, and the like), presence of measurement interference is suspected. Even in such a case, the case can be more suitably evaluated by accurately measuring the TSH activity value by the method of the present invention.

### Examples

### <Example 1: Preparation of Reporter Cell for TSH Measurement>

### (Purpose)

The purpose is to prepare a reporter cell for measuring the activity of thyroid-stimulating hormone (TSH).

### (Method and Results)

### (1) Preparation of vector for introducing TSH receptor gene

A gene expression vector for expressing TSH receptor gene in HEK293 cells (hereinafter referred to as "vector for introducing TSH receptor gene") was prepared by inserting a TSH receptor gene consisting of the base sequence of SEQ ID NO: 4, which encodes a human thyroid-stimulating hormone receptor (TSH receptor) consisting of the amino acid sequence of SEQ ID NO: 3, into a vector pcDNA3.1Zeo(+) (#V86020, Thermo Fisher Scientific Inc.).

### (2) Preparation of Reporter Cell

The vector for introducing TSH receptor gene, prepared in (1) above, was transfected to HEK293 cells into which pNL[NlucP/CRE/Hygro] (#CS186804, Promega Corporation) had been introduced, by the following method.

After culturing the NanoLuc-introduced HEK293 cells in Dulbecco's Modified Eagle Medium (DMEM) containing 10% fetal bovine serum (FBS), the number of cells was adjusted to 2.5 × 10⁵ cells/mL, and 2 mL of the cells were dispensed into each well of a 6-well plate and cultured under conditions of 37°C and 5% CO₂.

To 100 µL of MEM, 3 µL of the vector for introducing TSH receptor gene and 15 µL of PLUS REAGENT (#11514015, Thermo Fisher Scientific Inc.) were added and mixed, and then the resultant mixture was allowed to stand at room temperature for 15 minutes, followed by addition of 100 µL of MEM containing 4% Lipofectamine 2000 Transfection reagent (#11668027, Thermo Fisher Scientific Inc.). After mixing, the resultant mixture was allowed to stand at room temperature for 15 minutes, and then 800 µL of MEM was added and mixed.

The medium of the NanoLuc-introduced HEK293 cells after the culturing was removed by suction, and 1 mL of the MEM containing the vector for introducing TSH receptor gene and Lipofectamine 2000 Transfection reagent, prepared above, was dispensed, followed by culturing under conditions of 37°C and 5% CO₂ for 4 hours. Furthermore, the expanded cultured cells were preserved in liquid nitrogen in a cell preservation solution (Cell Banker).

Hereinafter, the NanoLuc-introduced HEK293 cell into which the vector for introducing TSH receptor gene prepared in this Example was introduced is referred to as "reporter cell for TSH measurement" or simply "reporter cell".

### <Example 2: Measurement of TSH activity using TSH standard solution>

### (Purpose)

An exemplary principle of measuring TSH activity by the reporter cell prepared in Example 1 is considered as follows. In the reporter cell, the TSH receptor expressed from the vector for introducing TSH receptor gene is expressed on the cell surface. When TSH binds to the TSH receptor to activate the TSH receptor, adenylate cyclase is activated via G protein, and transcription of a luciferase gene is induced upon stimulation of produced cAMP, so that luciferase protein is expressed in the reporter cell. The amount of the luciferase protein can be measured by quantifying luminescence intensity of luciferin based on luciferase activity. Accordingly, the amount of TSH can be quantified as luciferase activity.

In this Example, a TSH standard solution containing a standard concentration of TSH was added to the reporter cells to measure luminescence intensity based on luciferase activity, and a standard curve showing a relationship between TSH concentration and relative luminescence intensity was created.

### (Method and Results)

In a warm bath at 37°C, 1 mL of the reporter cell suspension prepared in Example 1 was thawed. To a 15 mL tube into which 9 mL of cell washing solution/diluent had been dispensed, the suspension was added and mixed gently, followed by centrifugation at 4°C and 1000 RPM for 5 minutes. As the cell washing solution/diluent, DMEM (No Glucose) with L-Gln, without Sodium Pyruvate, liquid (#09891-25, Nacalai Tesque, Inc.) containing 1% Penicillin-Streptomycin (10,000 U/mL; #15140-122, Thermo Fisher Scientific Inc.) was used. After removing the supernatant, 1 mL of the cell washing solution/diluent was added and mixed gently to prepare a cell suspension of 3 × 10⁵ cells/mL.

Into wells of a microplate for chemiluminescence, 40 µL of a standard solution containing TSH at a standard concentration (0 µIU/mL, 1 µIU/mL, 4 µIU/mL, 16 µIU/mL, 32 µIU/mL, or 128 µIU/mL), prepared using a TSH preparation (WHO International Standard Thyroid Stimulating Hormone, Human, for Immunoassay; NIBSC code 81/565) purchased from National Institute for Biological Standards and Control (NIBSC) was dispensed. To each well, 40 µL of the cell suspension prepared above was added and mixed gently, and then the plate was covered with a plate cover and allowed to stand under conditions of 37°C and 5% CO₂ for 4 hours. Into each well, 80 µL of Nano-Glo (registered trademark) luminescent substrate (Nano-Glo (registered trademark) Luciferase Assay System, #N1130, Promega Corporation) was dispensed and shaken at room temperature (20°C to 25°C) for 5 minutes, and then luminescence intensity of all wells was measured for 0.5 seconds with a chemiluminescence microplate reader (Tristar LB941, Berthold Technologies) to measure luciferase activity.

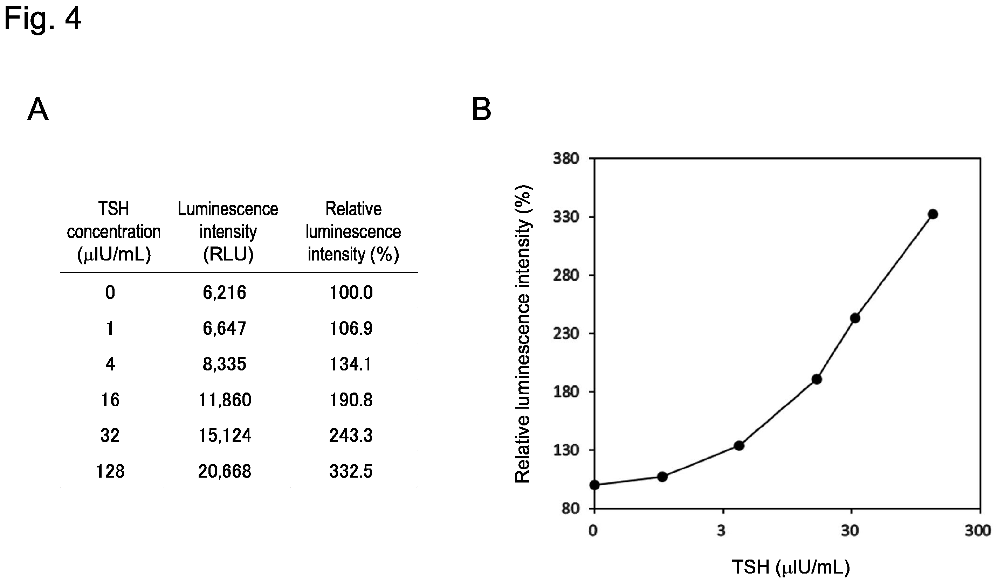

A ratio (%) of luminescence intensity of the standard solution of each TSH concentration to an average value of luminescence intensity of the standard solution with a TSH concentration of 0 µIU/mL was calculated and used as relative luminescence intensity of each TSH standard solution. The results are shown in Figure 4A. Furthermore, a standard curve was created by plotting the TSH concentration of each standard solution on the horizontal axis and the relative luminescence intensity of each standard solution on the vertical axis (Figure 4B). The obtained standard curve has revealed that TSH activity can be quantified in a range of at least 0.5 µIU/mL to 128 µIU/mL.

### <Example 3: Comparison between TSH activity measurement using reporter cell and immunological measurement method>

### (Purpose)

The purpose is to compare the results of measuring TSH activity in serum samples using reporter cells with the results of measuring the same serum samples by an immunological measurement method based on an antigen-antibody reaction.

### (Method and Results)

### (1) Serum sample

TSH was measured in serum obtained from 284 patients comprising overt hypothyroidism and subclinical hypothyroidism. Serum samples were inactivated by being allowed to stand in a thermostat bath set at 56°C for 30 minutes, returned to room temperature (20°C to 25°C), and then diluted 2-fold with a cell washing solution/diluent. The resultant samples were used for the following measurement.

### (2) TSH activity measurement using reporter cell

By the same method as in Example 2, the serum sample of (1) above was dispensed into wells of a microplate for chemiluminescence, and 40 µL of cell suspension containing reporter cells was added to each well and mixed gently. Then, the plate was covered with a plate cover and allowed to stand under conditions of 37°C and 5% CO₂ for 4 hours. Into each well, 80 µL of Nano-Glo (registered trademark) luminescent substrate was dispensed and shaken at room temperature (20°C to 25°C) for 5 minutes, and then luminescence intensity of all wells was measured with a chemiluminescence microplate reader to measure luciferase activity.

### (3) Quantification of TSH by immunological measurement method

TSH in the serum sample of (1) above was quantified using an immunological measurement method. Specifically, TSH was quantified using Elecsys (registered trademark) reagent TSH (Cobas) of a thyroid-stimulating hormone kit according to the attached instructions.

### (4) Comparison of quantification results

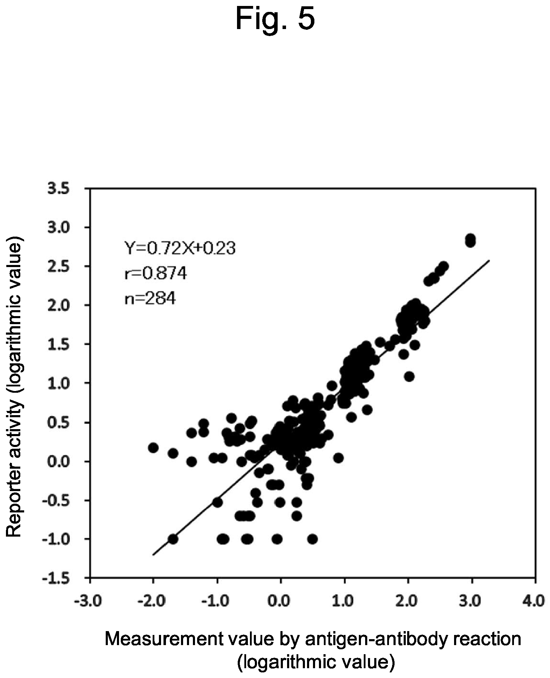

Figure 5 shows the results of logarithmically converting the measurement values obtained in (2) and (3) above and plotting the values on the vertical axis and the horizontal axis, respectively. A correlation (correlation coefficient r = 0.874) was observed between the two as a whole, and it has been revealed that the correlation is strong in a high concentration range (range of from 10 to 700 µIU/mL in TSH activity value). On the other hand, it has been revealed that the correlation is weak in a low concentration range (range of from 0.1 to 10 µIU/mL in TSH activity value). From these results, it has been demonstrated that the TSH concentration measured by the immunological measurement method does not accurately reflect the activity of TSH, particularly in the low concentration range.

### <Example 4: TSH activity measurement in serum samples of patients with subclinical hypothyroidism and patients with overt hypothyroidism>

### (Purpose)

The purpose is to measure TSH activity in serum samples of patients with subclinical hypothyroidism and patients with overt hypothyroidism using reporter cells.

### (Method and Results)

TSH activity in serum obtained from subjects with normal thyroid function (267 persons), patients with subclinical hypothyroidism (89 persons), and patients with overt hypothyroidism (121 persons), classified based on diagnosis by a doctor, was measured using reporter cells.

Inactivated serum samples were prepared by the same method as in "(1) Serum sample" of Example 3, and luciferase activity was measured by the same method as in "(2) TSH activity measurement using reporter cell" of Example 3. Simultaneously, luciferase activity was measured for TSH standard solutions by the same method as in Example 2 to create a standard curve, and TSH activity values (µIU/mL) were calculated for each serum sample on the basis of the luciferase activity and the standard curve.

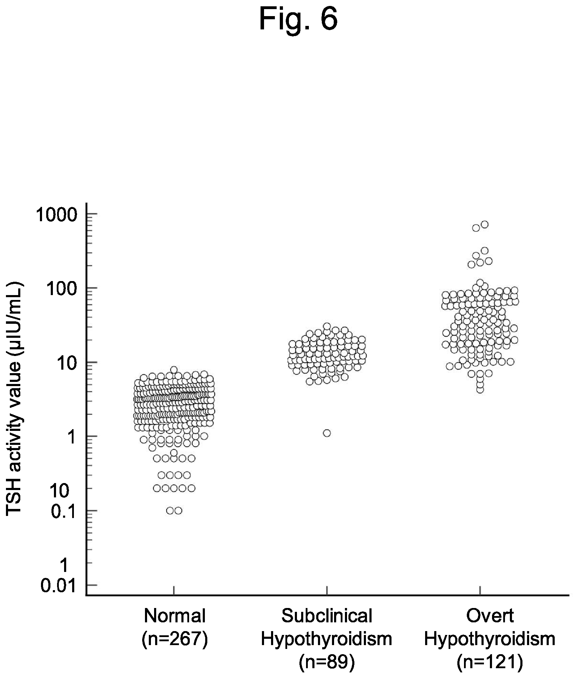

Figure 6 shows the results of plotting TSH activity values for each group. Significant differences in TSH activity values were detected between healthy subjects, and patients with subclinical hypothyroidism and patients with overt hypothyroidism (non-parametric comparison was performed for all pairs, and significant differences were detected by a Steel-Dwass test). This result indicates that a healthy subject and a patient with subclinical hypothyroidism, which were difficult to distinguish by a conventional technique, can be distinguished by luciferase activity measurement using the reporter cell of the present invention.

All publications, patents, and patent applications cited herein are incorporated herein by reference in their entirety.

## Claims

1. A reporter cell for measuring thyroid-stimulating hormone (TSH) activity, wherein:
a TSH receptor gene encoding a TSH receptor or an active fragment thereof and a reporter gene encoding a reporter protein are introduced into the reporter cell, and
the reporter gene is placed under the control of a promoter whose promoter activity is regulated depending on the activity of the TSH receptor or the active fragment thereof.

2. The reporter cell of claim 1, wherein the promoter comprises a cAMP response element.

3. The reporter cell of claim 1, wherein the reporter protein is a luminescent protein, a fluorescent protein, or an enzyme protein.

4. The reporter cell of claim 1, wherein the reporter cell is a mammalian cell.

5. The reporter cell of claim 4, wherein the mammalian cell is an HEK293 cell, a HeLa cell, an NIH3T3 cell, a CHO cell, a COS cell, or a Vero cell.

6. A kit for measuring TSH activity, comprising the reporter cell of claim 1.

7. The kit for measuring TSH activity of claim 6, further comprising a TSH standard solution containing a standard concentration of TSH.

8. The kit for measuring TSH activity of claim 6 or 7, wherein
the reporter protein is a luminescent protein, and
the kit further comprises a luminescent substrate for the luminescent protein.

9. A method for measuring the activity of thyroid-stimulating hormone (TSH) in a sample, comprising:
a culturing step of culturing a reporter cell in a medium containing the sample; and
a reporter activity measurement step of measuring the activity of the reporter protein derived from the reporter cell after the culturing, wherein
a TSH receptor gene encoding a TSH receptor or an active fragment thereof and a reporter gene encoding a reporter protein are introduced into the reporter cell, and
the reporter gene is placed under the control of a promoter whose promoter activity is regulated depending on the activity of the TSH receptor or the active fragment thereof.

10. The method of claim 9, wherein
the reporter protein is a luminescent protein, and
the reporter activity measurement step comprises adding a luminescent substrate for the luminescent protein to the reporter cell and measuring the luminescence intensity of the luminescent substrate.

11. A method for determining hypothyroidism or hyperthyroidism, comprising:
a culturing step of culturing a reporter cell in a medium containing a body fluid derived from a subject;
a reporter activity measurement step of measuring the activity of a reporter protein derived from the reporter cell after the culturing to produce a reporter activity value; and
a determination step of determining that the subject has a overt hypothyroidism or an subclinical hypothyroidism when the reporter activity value or a TSH activity value calculated based thereon is higher than an upper reference value for TSH, and determining that the subject has a overt hyperthyroidism or an subclinical hyperthyroidism when the reporter activity value or the TSH activity value is lower than a lower reference value for TSH, wherein
a TSH receptor gene encoding a TSH receptor or an active fragment thereof and a reporter gene encoding a reporter protein are introduced into the reporter cell, and
the reporter gene is placed under the control of a promoter whose promoter activity is regulated depending on the activity of the TSH receptor or the active fragment thereof.

12. A method for determining hypothyroidism or hyperthyroidism, comprising:
a culturing step of culturing a reporter cell in a medium containing a body fluid derived from a subject;
a reporter activity measurement step of measuring the activity of a reporter protein derived from the reporter cell after the culturing to produce a reporter activity value;
an FT4/FT3 measurement step of measuring the concentrations of free thyroxine (FT4) and free triiodothyronine (FT3) in the body fluid derived from the subject to produce an FT4 measurement value and an FT3 measurement value; and
a determination step which comprises the following (a) to (d), based on the reporter activity value or a TSH activity value calculated based thereon, and the FT4 measurement value and the FT3 measurement value:
(a) determining that the subject has a overt hypothyroidism when the reporter activity value or the TSH activity value is higher than the upper reference value for TSH and at least one of the FT4 measurement value and the FT3 measurement value is lower than a lower reference value thereof;
(b) determining that the subject has an subclinical hypothyroidism when the reporter activity value or the TSH activity value is higher than the upper reference value for TSH and the FT4 measurement value and the FT3 measurement value fall within their respective lower and upper reference values;
(c) determining that the subject has an subclinical hyperthyroidism when the reporter activity value or the TSH activity value is lower than a lower reference value for TSH and the FT4 and FT3 measurement values fall within their respective lower and upper reference values;
(d) determining that the subject has a overt hyperthyroidism when the reporter activity value or the TSH activity value is lower than the lower reference value for TSH and at least one of the FT4 measurement value and FT3 measurement value is higher than an upper reference value thereof, wherein
a TSH receptor gene encoding a TSH receptor or an active fragment thereof and a reporter gene encoding a reporter protein are introduced into the reporter cell, and
the reporter gene is placed under the control of a promoter whose promoter activity is regulated depending on the activity of the TSH receptor or the active fragment thereof.

13. The method of claim 11 or 12, wherein, in the determination step, the TSH activity value is calculated based on the reporter activity value and a standard reporter activity value which is produced based on a standard concentration of TSH.

14. The method of claim 11 or 12, wherein
the reporter protein is a luminescent protein, and
the reporter activity measurement step comprises adding a luminescent substrate for the luminescent protein to the reporter cell and measuring the luminescence intensity of the luminescent substrate.

15. The method of claim 11 or 12, wherein the body fluid is blood, serum, or plasma.

16. The method of claim 11 or 12, wherein the lower reference value for TSH is 0.1 to 1.0 µIU/mL and/or the upper reference value for TSH is 4.0 to 9.0 µIU/mL.

17. The method of claim 11 or 12, wherein the lower reference value for FT4 is 0.9 ng/dL, and the upper reference value for FT4 is 1.7 ng/dL, and/or the lower reference value for FT3 is 2.3 pg/mL, and the upper reference value for FT3 is 4.0 pg/mL.

18. The method of claim 11 or 12, wherein the subject is pregnant or trying to conceive.

19. The kit for measuring TSH activity of claim 6 or 7, for use in the method of claim 11 or 12.
